# EUROPEAN PATENT SPECIFICATION

(11) **EP 1 553 187 B1**
(45) Date of publication and mention of the grant of the patent: **02.01.2008**
(21) Application number: 03723278.2
(22) Date of filing: 08.05.2003
(51) Int. Cl.: C12Q 1/26

(54) **METHOD FOR TRAPPING INTERMEDIATES FORMED BY THE REACTION BETWEEN AN OXIDOREDUCTASE AND ITS SUBSTRATES**
VERFAHREN ZUM AUFSAMMELN VON DURCH DIE REAKTION EINER OXIDOREDUKTASE MIT IHREN SUBSTRATEN GEBILDETEN ZWISCHENPRODUKTEN
PROCEDE DE CAPTURE D'INTERMEDIAIRES FORMES PAR LA REACTION ENTRE UNE OXYDOREDUCTASE ET SES SUBSTRATS

(30) Priority: 28.08.2002 JP 2002248909
(43) Date of publication of application: 13.07.2005
(73) Proprietor: Japan Science and Technology Agency, Kawaguchi-shi Saitama 332-0012 (JP)
(72) Inventor: OGURA, Takashi, Ako-gun, Hyogo 678-1205 (JP); KUROIWA, Shigeki 503 Yamamoto Gurandohaitsu, Kyoto-shi, Kyoto 607-8163 (JP); YOSHIKAWA, Shinya, Ako-gun, Hyogo 678-1205 (JP)
(74) Representative: Wilson Gunn
(86) International application number: PCT/JP2003/005797
(87) International publication number: WO 2004/020657

(56) References cited:
- JP-A- 6 271 519
- JP-A- 8 092 179
- RYLE M J ET AL: "Nitrogenase reduction of carbon disulfide: freeze-quench EPR and ENDOR evidence for three sequential intermediates with cluster-bound carbon moieties." BIOCHEMISTRY. 8 FEB 2000, vol. 39, no. 5, 8 February 2000 (2000-02-08), pages 1114-1119, XP002397371 ISSN: 0006-2960
- SCHUNEMANN^A V ET AL: "Intermediates in the reaction of substrate-free cytochrome P450cam with peroxy acetic acid" FEBS LETTERS, ELSEVIER, AMSTERDAM, NL, vol. 479, no. 3, 18 August 2000 (2000-08-18), pages 149-154, XP004337483 ISSN: 0014-5793
- STACH P. ET AL.: 'Bacterial cytochrome C nitrite reductase: new structural and functional aspects' J. INORG. BIOCHEM. vol. 79, no. 1-4, 2000, pages 381 - 385, XP002970430
- POWERS L. ET AL.: 'Multiple structures and functions of cytochrome oxidase' J. INORG. BIOCHEM. vol. 23, no. 3-4, 1985, pages 207 - 217, XP002970431

## Description

### Field of the Invention

The present invention relates to a method for trapping the intermediates of reaction between oxidation-reduction enzymes (oxidoreductase), such as cytochrome-c oxidase, and their substrates.

### Description of the Prior Art

Since it is important for the elucidation of the reaction mechanism to trap the reaction intermediates of oxidation-reduction processes catalyzed by an oxidoreductase, such as cytochrome-c oxidase, various attempts at trapping have been tried.

For example, there has been reported a method for trapping reaction intermediates generated by oxygen. In the method, carbon monoxide was used as an inhibitor of reduced form of cytochrome-c oxidase. After the enzyme was inactivated by carbon monoxide, oxygen was added at around -25degree C. Then, the mixture was frozen at a further lower temperature and was irradiated with light. The inhibitor, carbon monoxide, was dissociated by the irradiation. Thus, the reaction intermediate including oxygen was trapped (Chance, B., Saronio, C., Leigh, Jr., J.S., Ingledew, W.J., and King, T.E. (1978) Biochem. J., 171, 787-798). In this case, the possibility that the inhibitor might induce side reactions cannot be excluded. Therefore, the method cannot be applied to those enzymes, wherein appropriate inhibitors are not found. When the enzyme is a protein crystal, it accompanies further disadvantage in that rapid mixing with substrates is difficult.

Furthermore, it is reported that photoreduction of cytochrome-c oxidase was achieved by ruthenium complex, which release electrons by light-irradiation at room temperature (Scott, R.A. and Gray, H.B. (1980) J. Am. Chem. Soc., 102, 3219-3224). However, attempts to trap the reaction intermediates, using this complex in frozen state as described in the previous reference, accompany a problem that the yield of photoreduction is very low because of the absence of molecular diffusion at a low temperature in contrast to a room temperature.

Previously, the intermediates of the reaction between the enzyme and the substrates have been discussed based on the results of time-resolved spectroscopy. Furthermore, the protein structure of the reaction intermediates has been inferred from the X-ray crystallographical analysis of protein crystals before and after the reaction.

### Problems to be solved by the Invention

The present invention provides a method for trapping reliably the reaction intermediates of an oxidoreductase. The method involves a use of a photoinduced reducing agent, which releases an electron by photo-irradiation, and sufficient reduction of the oxidoreductase, such as cytochrome-c oxidase, by the released electrons at a low temperature, wherein the aqueous solution containing the oxidoreductase and a substrate is in a frozen state. Furthermore, the present invention provides a method for trapping the reaction intermediates generated in the enzymes in a crystalline state without using inhibitors of the enzyme as used previously.

### Means to solve the Problems

To solve the above problems, the inventors initiate the reaction by mixing enzyme solution containing substrates at a low temperature, by using a reagent releasing electrons by light-irradiation and by irradiating the solution with light to reduce the enzyme. Since the reaction is performed at a low temperature, the diffusion of reactants is limited and the yield of the enzyme reduction is low. However, the inventors discovered an ideal condition to reduce efficiently the enzyme by the use of amine-type electron donors to supply electrons to photochemical reactants. In this way, the inventors succeeded in trapping reliably the reaction intermediates of an oxidoreductase.

To exclude possible side reactions by inhibitors and to apply the method to those enzymes, for which no appropriate inhibitors are found, in the method of the present invention, the reaction between the enzyme and the substrates is initiated at a low temperature without using inhibitors, and the reaction intermediates are trapped at a low temperature. There is no need to mix rapidly the enzyme with the substrates, since the reaction does not take place when starting mixing. Therefore, the method is applicable to protein crystals and opens a route for X-ray crystallographic analysis of the reaction intermediates.

More specifically, the present invention is a method for trapping reaction intermediates of an oxidoreductase comprising the steps of:
(the first step) dissolving an oxidoreductase, a photoinduced reducing agent that releases electrons by light-irradiation, amine-type electron donor and a substrate for said oxidoreductase in water and mixing these;
(the second step) cooling the mixture prepared in the first step to 70∼270K to be frozen;
(the third step) irradiating the frozen mixture prepared in the second step at 70~270K with a light in a wavelength region including the absorbing wavelength of said photoinduced reducing agent; and
(the fourth step) raising the temperature of the frozen mixture prepared in the third step to the temperature that is 80~270K and is higher than the temperature of the third step.

### Brief Description of the Drawings

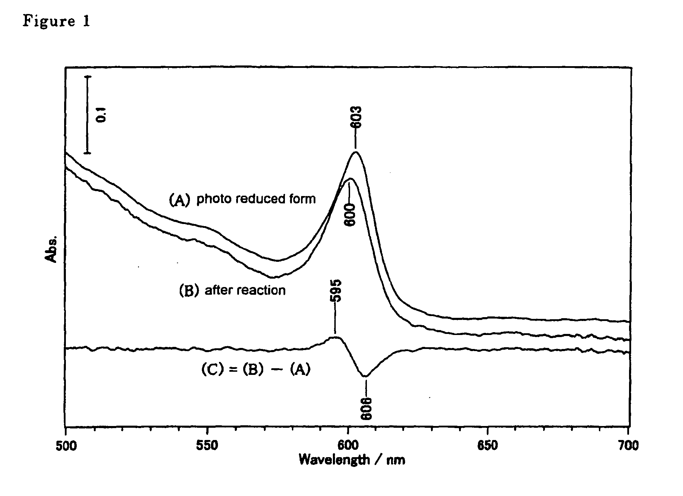

Figure 1 shows low temperature absorption spectra of cytochrome-c oxidase.

### Detailed Description of the Invention

In the first step, an oxidoreductase, a photoinduced reducing agent which release electrons by light-irradiation, amine-type electron donor and substrates for said oxidoreductase are dissolved in water. Among the reactants, gas reactant as a substrate could be bubbled through the solution after all other reactants have been mixed.

An oxidoreductase is an enzyme, which acts as a catalyst in a certain oxidation-reduction reaction, and belongs to the EC1 group, one of the main groups of enzymes, which was defined by an enzyme board of International Union of Biochemistry (IUB). This enzyme group includes alcohol dehydrogenase, hydrogenase, nitrogen monoxide reductase, denitrification enzymes (nitric acid reductase, nitrous acid reductase, oxidized nitrogen reductase, nitrogen suboxide reductase), P450 super-family (P450nor, P450scc, P450cam, etc.), quinol oxigenase, mitochondrial inner membrane electron transport enzymes (complex I, complex II, cytochrome bc₁ complex, cytochrome-c oxidase, etc.), photosynthesis electron transport enzymes (cytochrome b₆f complex, Fd-NADP⁺ reductase, etc.), oxigenases (tryptophan 2,3-dioxigenase, phenylalanine 4 monoxigenase, etc.) and hydroperoxidases (catalase, peroxidase). The group also includes said enzymes containing point mutations of amino acids. The concentration of the oxidoreductase in an aqueous solution is about 1 µM∼10 mM.

The photoinduced reducing agent, which releases electrons by photo-irradiation, includes transition metal complexes such as ruthenium complexes ([Ru(bipyridine)₃]²⁺, [Ru(NH₃)₆]²⁺, etc.), hydrocarbons (perylene and its derivatives, pyrene and its derivatives, etc.), various dyes (porphyrines (without Zn, Mg or metal ions), pH indicators (methylene blue, acridine orange, etc.), methylviologene, etc.).

Among them, [Ru(bipyridine)₃]²⁺ has absorption maxima at 452 nm and 426 nm. Therefore, the complex is excited by light-irradiation covering this wavelength region. The amount of photoinduced reducing agents in water needs generally about 2 ∼ 10 times more than the reduction equivalence of the enzyme; or more than that because of inefficient yield of photoreduction at a low temperature. Finally, the concentration of the complex is about 1 µ M∼100 mM.

The substrate is a compound, which is subject to a catalytic action by said oxidoreductase and changes to a reduced state by accepting electrons. This substrate includes oxygen, in the case of quinol oxidase and cytochrome-c oxidase; nitrogen monoxide, in the case of nitrogen monoxide reductase; oxygen, carbon monoxide and organic compounds (for example, cholesterol and champhor), in the case of P450 superfamily. Furthermore, an electron acceptor other than physiological substrate of an enzyme can be used as a substrate. The concentration of substrates in an aqueous solution is about 1 µM∼20 mM.

An amine-type electron donor is an amine compound having amino group, imino group, hydrazine group and the like, which can regenerate by supplying electrons to the photoinduced reducing agents that is in an oxidized form after releasing electrons. The amine-type electron donor prevents a reverse reaction, wherein electrons transferred to substrates or enzymes from electron donors return to the photoinduced reducing agent again, and is stable enough to be abstracted its electrons by oxidized form of photoinduced reducing agents. The amine-type electron donor includes, for example, ethylenediamine tetra-acetic acid, triethanol amine, L-cysteine, aniline, N, N-dimethylaniline and the like.

The combination of some of them also can be used as the reducing agent. The concentration of amine-type electron donor in the aqueous solution is about 1 mM-100 mM.

pH buffer agents, solubilizing agents and other reagents may be added to the aqueous solution if necessary.

Phosphate buffer or Good buffer can be used as pH buffer agents and they keep the enzyme solution at an appropriate pH (pH 1-14), wherein said oxidoreductase functions normally. The concentration of pH buffer is preferably used at higher concentration than that of photoinduced reducing agents, in order to moderate the pH changes during the reaction of the photoinduced reducing agents. The concentration of pH buffer is usually about 1∼200 mM.

When said oxidoreductases are not soluble proteins in water, surfactants (n-decyl-β-D-maltopyranoside, n-dodecyl-*β-*D-maltopyranoside, cholic acid, triton X-100, etc.) can be used as solubilizing agents. Their concentrations are 0~1% (W/V).

Additionally, it is desirable that the concentration of each reactant is controlled appropriately to keep the oxidoreductase normally functional.

In the second step, the mixture prepared in the first step is cooled and frozen. The temperature is 70-270K, wherein the solution is in frozen state and photoreduction is possible.

The temperature is preferably maintained at lower than the temperature, at which substrates start to diffuse in the mixture (hereinafter, the temperature is referred to as "diffusion onset temperature") and is simply liquid nitrogen temperature (77K). The diffusion onset temperature is characteristic to a substrate and constant in the present invention. The diffusion onset temperature is explained in the example and it is 170K and 140∼170K for oxygen and carbon monoxide, respectively.

In the third step, the frozen mixture is irradiated with light. Electrons are released from the photoinduced reducing agents in their excited state and are accepted by the oxidoreductase, which are changed to a reduced state. The temperature in this state could be the same to that of the second step. However, it is preferably lower than the diffusion onset temperature not to start the reaction with the substrates. Furthermore, the temperature is more preferably higher temperature in this region to enhance the rate of the reduction reaction. Therefore, the temperature in this step is preferably lower than the diffusion onset temperature and more preferably 5~20K lower than the diffusion onset temperature.

In the fourth step, the temperature is raised more than that of the previous step in order to allow to react the reduced form of oxidoreductase with the substrate and to produce reaction intermediates. If the temperature is raised too high, the reaction proceeds so rapidly that the lifetime of the intermediates might be short. Therefore, the temperature is more than the diffusion onset temperature but a lower temperature as much as possible. Accordingly, the temperature in this step is higher than that of the previous step, preferably higher than the diffusion onset temperature but lower than 270K, more preferably between the diffusion onset temperature and the diffusion onset temperature plus 50K, much more preferably between the diffusion onset temperature and the diffusion onset temperature plus 30K and most preferably the diffusion onset temperature.

In the method of the present invention, it is possible to add the fifth step, wherein the frozen mixture prepared in the fourth step is kept at a lower temperature than the diffusion onset temperature to maintain the reaction intermediates. Usually it is liquid nitrogen temperature (77K), which is the simplest way.

### Effects of the Invention

(1) The method of the present invention can be applied to protein engineering in the following way.
   The techniques to trap reaction intermediates at a low temperature will be important not only for existing enzyme proteins but also for future artificial enzymes designed for functioning oxidation-reduction reaction.
   By means of said technique, it will become easier to determine the structure of reaction intermediates by X-ray crystallographic analysis as a way to assay or to evaluate the function of enzymes. According to prior art, the enzymes that can fasten reaction intermediates are limited to those that can utilize the inhibitors or altered substrates and those that can initiate photochemical reactions by themselves at low temperatures. Other enzymes need to be modified so that the reaction is stopped at the intermediate stage of the reaction. However, there remains the possibility that the modified enzyme proteins may change their structure. Besides, appropriately engineered enzyme proteins are not always available. In such a situation, only a spectroscopic method remains to be discussed. However, a spectroscopic method cannot always get the information on every detailed structural change of the enzyme proteins under the reaction.
   The present invention is a method for trapping the reaction intermediates without using inhibitors or engineered enzyme proteins and is applicable to a broad range of enzymes. Furthermore, examination of each step of a enzyme reaction will be possible by developing a photochemical reagents with higher sensitivity to light; by developing a reagents, wherein photochemical reagents and electron donor are combined; or by donating a single electron to the enzyme by a pulse of light at a low temperature and by inducing a single step of reactions by a single electron.
(2) Furthermore, the method of the present invention can be applicable to a low temperature oxygen detection apparatus. Usually, an oxygen electrode is used to measure a concentration of oxygen in a solution, but it is not applicable to a frozen solution. Using enzymes whose substrate is oxygen, it is possible to design an apparatus, by which oxygen concentration can be measured in its frozen state.
(3) Moreover, the method of the present invention can be applied to an apparatus to detect small amount of nitrogen monoxide. Usually, nitrogen monoxide generated *in vivo* is unstable. However, it is possible to detect spectroscopically small amount of nitrogen monoxide by mixing rapidly biological samples, nitrogen monoxide reductase (oxidized form) and photochemical reagents; then by freezing the mixture; then by reducing photochemically the frozen sample; and then by allowing to react the irradiated mixture with nitrogen monoxide by raising the temperature.

The following examples illustrate this invention, however, these are not constructed to limit the scope of the invention.

### Example 1

In this example, on the basis of the following steps, the reaction intermediates are trapped, which are produced during reduction of the substrate (oxygen) by cytochrome-c oxidase.
(1) Dissolving cytochrome-c oxidase (prepared according to the method described in Yoshikawa et al. (1977) Journal of Biological Chemistry, 252,5498-5508) in water at a concentration of 20 µM in 50 mM phosphate buffer (pH 6.8) containing 0.2 % n-decyl-β-D-maltopyranoside (purchased from Anatrace).
(2) Saturating the solution of (1) with oxygen gas by bubbling.
(3) Adding 2-sodium-ethylenediaminetetra-acetate (Wako, Co) and aniline (Kanto Chem. Co.) at 20 mM and 5 mM, respectively, to the solution of (2).
(4) After adding tris (2,2'-bipyridine) dichlororuthenium (II) (Obtained from Sigma Co.) at a concentration of 100 µM to the solution of (3) in the dark, freezing it in liquid nitrogen.
(5) Raising the temperature of the solution of (4) to 140K and irradiating the solution with a 150 W halogen lamp for 4 hrs (Wavelength region: 400∼800 nm; the integrated energy of light: 1 x 10²~5 x 10⁶ J). The absorption spectrum of the solution is shown in Fig. 1. As shown in (A), there is a small absorption peak around 603 nm and there is no absorbance due to the reagents used. The peak around 603 nm corresponds to the absorption peak due to the reduced form of cytochrome-c oxidase. The ruthenium complex is photo-excited by light-irradiation, released electron and transferred the electron to cytochrome-c oxidase. Then, the cytochrome-c oxidase is changed to a reduced form.
   Incidentally, since the ruthenium complex is a strong oxidant after releasing an electron, it will start a reverse reaction, wherein the complex withdraws an electron from the reduced form of cytochrome-c oxidase, and the forward reaction does not proceed further. In the case of the reaction in solution at room temperature, an appropriate electron-donor added to the solution can re-reduce the oxidized form of ruthenium complex. However, in the case of the reaction at a low temperature, wherein the solution is in frozen state and the rate of molecular diffusion is low, it is very difficult to transfer an electron to the oxidized form of ruthenium complex. In the present invention, the inventors resolved the problem by electron-donors of amines (a mixture of ethylenediaminetetra-acetic acid and aniline). As the result, 100% of photo-reduction of cytochrome-c oxidase was accomplished.
(6) Allowing the reduced form of cytochrome-c oxidase to react with oxygen at 180K for 5 hrs. As shown in Fig. 1 (B), a shift of absorption maximum is observed. Fig. 1 (c) shows the calculation result obtained by subtracting the spectrum of (A) from that of (B).

As the temperature of the solution of (5) is raised to 180K, oxygen molecules start to diffuse gradually and bind to a heme iron of heme a₃ that is an active center of the reduced form of cytochrome-c oxidase.

As the result, Fe³⁺-O₂⁻ (Fe³⁺ is a heme iron of heme a₃), an oxygenated form of reaction intermediate, is generated. The spectrum difference between before and after the reaction with oxygen shown in Fig. 1 (C) is identical to those reported for the oxygenated form of the reaction intermediate (Biochem, J., 171, 787-798 (1978), T. Ogura et al., (1990) J. Am. Chem. Soc., 112, 5630-5631).

In Figure 1, the calculation of difference spectrum of reaction intermediate (B) and reduced form of the oxidoreductase (A) reveals an increase of absorption around 595 nm and a decrease of absorption around 606 nm (C). These changes are not observed under anaerobic condition, i.e. the reaction without oxygen. The result indicates that the absorbance around 606 nm before the reaction shifted to around 595 nm after the reaction with oxygen. This means that the reduced form of heme a₃ is changed to something by oxygen.

According to Chance et al. (Biochem. J., 171, 787-798 (1978)), when the oxygenated form of the reaction intermediate (compound A in the paper) is formed, an increase of absorption around 591 nm and a decrease an absorption around 611 nm are observed. Considering the difference in the experimental conditions (difference in enzyme preparation, presence or absence of a solubilizing agent, difference in reaction temperature and difference in the amount of reaction intermediates), it is concluded that an oxygenated form of reaction intermediate was produced.

## Claims

1. A method for trapping reaction intermediates of an oxidoreductase comprising the steps of:
(step 1) dissolving an oxidoreductase, a photoinduced reducing agent that releases electrons by light-irradiation, amine-type electron donor and a substrate for said oxidoreductase in water and mixing these;
(step 2) cooling the mixture prepared in the first step to 70∼270K to be frozen;
(step 3) irradiating the frozen mixture prepared in the second step at 70∼270K with a light in a wavelength region including the absorbing wavelength of said photoinduced reducing agent; and
(step 4) raising the temperature of the frozen mixture prepared in the third step to the temperature that is 80~270K and is higher than the temperature of the third step.

2. The method as in claim 1 wherein the mixture prepared in the first step is cooled to the temperature lower than the temperature at which the substrate starts to diffuse in said mixture in the second step, hereinafter referred to as "diffusion onset temperature", the frozen mixture prepared in the second step is irradiated with a light at a temperature lower than the diffusion onset temperature in the third step; and the temperature of the frozen mixture prepared in the third step is raised to a temperature higher than the diffusion onset temperature but lower than 270K in the fourth step.

3. The method as in claim 2 wherein the frozen mixture prepared in the second step is irradiated with a light at a temperature which is 5∼20K lower than the diffusion onset temperature in the third step; and the temperature of the frozen mixture prepared in the third step is raised to a temperature between the diffusion onset temperature and the diffusion onset temperature plus 50K but lower than 270K in the fourth step,.

4. The method as in any one of claims 1 to 3 further comprising (fifth step) cooling the frozen mixture prepared in the fourth step to a temperature lower than the diffusion onset temperature.

## Patentansprüche

1. Ein Verfahren zum Einfangen von Reaktionszwischenprodukten einer Oxidoreduktase umfassend die Schritte von:
(Schritt 1) Auflösen einer Oxidoreduktase, eines fotoinduzierten reduzierenden Agens, das Elektronen durch Lichtbestrahlung freisetzt, eines Amintyp-Elektronendonors sowie eines Substrates für besagte Oxidoreduktase, in Wasser und Mischen dieser Komponenten;
(Schritt 2) Abkühlen der Mischung, hergestellt im ersten Schritt, auf 70-270K, zum Gefrieren;
(Schritt 3) Bestrahlen der gefrorenen Mischung, hergestellt in dem zweiten Schritt bei 70-270K mit Licht in einer Wellenlängenregion, welche die absorbierende Wellenlänge von besagtem fotoinduzierten reduzierenden Agens einschließt; und
(Schritt 4) Anheben der Temperatur der gefrorenen Mischung, hergestellt in dem dritten Schritt, auf die Temperatur die 80-270K ist und höher ist, als die Temperatur des dritten Schrittes.

2. Das Verfahren gemäß Anspruch 1, wobei die Mischung, die in dem ersten Schritt hergestellt wird, in dem zweiten Schritt abgekühlt wird auf die Temperatur, die geringer ist als die Temperatur, bei welcher das Substrat in besagter Mischung zu diffundieren beginnt, wobei die Temperatur hier im folgenden als "Diffusionsbeginntemperatur" bezeichnet wird; die gefrorene Mischung, die in dem zweiten Schritt hergestellt wird, in dem dritten Schritt bestrahlt wird mit Licht bei einer Temperatur, welche geringer ist als Diffusionsbeginntemperatur; und die Temperatur der gefrorenen Mischung, hergestellt in dem dritten Schritt, in dem vierten Schritt angehoben wird auf eine Temperatur von mehr als die Diffusionsbeginntemperatur, aber geringer als 270 K.

3. Das Verfahren gemäß Anspruch 2, wobei die gefrorene Mischung, die in dem zweiten Schritt hergestellt wird, in dem dritten Schritt bestrahlt wird mit Licht bei einer Temperatur, welche 5-20K niedriger ist als die Diffusionsbeginntemperatur, und die Temperatur der gefrorenen Mischung, hergestellt in dem dritten Schritt, in dem vierten Schritt erhöht wird auf eine Temperatur zwischen der Diffusionsbeginntemperatur und der Diffusionsbeginntemperatur +50K, jedoch geringer ist als 270K.

4. Das Verfahren, wie in irgendeinem der Ansprüche 1 bis 3, welches desweiteren umfaßt:
(Schritt 5) Abkühlen der gefrorenen Mischung, hergestellt in dem vierten Schritt auf eine Temperatur, welche geringer ist als die Diffusionsbeginntemperatur.

## Revendications

1. Procédé pour piéger des intermédiaires de réaction d'une oxydoréductase comprenant les étapes de :
(étape 1) dissoudre une oxydoréductase, un agent réducteur photo-induit qui libère des électrons par irradiation de lumière, un donneur d'électrons de type amine et un substrat pour ladite oxydoréductase dans de l'eau et mélanger ceux-ci ;
(étape 2) refroidir le mélange préparé à la première étape à 70-270°K pour le congeler ;
(étape 3) irradier le mélange congelé préparé à la deuxième étape à 70-270°K par une lumière dans une région de longueur d'onde incluant la longueur d'onde d'absorption dudit agent réducteur photo-induit ; et
(étape 4) élever la température du mélange congelé préparé à la troisième étape jusqu'à la température qui est de 80-270°K et est supérieure à la température de la troisième étape.

2. Procédé selon la revendication 1, dans lequel le mélange préparé à la première étape est refroidi à la température inférieure à la température à laquelle le substrat commence à diffuser dans ledit mélange à la deuxième étape, appelée ci-dessous "température de début de diffusion" ; le mélange congelé préparé à la deuxième étape est irradié par une lumière à une température inférieure à la température de début de diffusion à la troisième étape ; et la température du mélange congelé préparé à la troisième étape est élevée à une température supérieure à la température de début de diffusion mais inférieure à 270°K à la quatrième étape.

3. Procédé selon la revendication 2, dans lequel le mélange congelé préparé à la deuxième étape est irradié par une lumière à une température qui est de 50-20°K inférieure à la température de début de diffusion à la troisième étape ; et la température du mélange congelé préparé à la troisième étape est élevée à une température entre la température de début de diffusion et la température de début de diffusion plus 50°K mais inférieure à 270°K à la quatrième étape.

4. Procédé selon l'une quelconque des revendications 1 à 3, comprenant de plus (cinquième étape) le refroidissement du mélange congelé préparé à la quatrième étape à une température inférieure à la température de début de diffusion.
